# FASCICULE DE BREVET EUROPEEN

(11) **EP 2 143 375 B1**
(45) Date de publication et mention de la délivrance du brevet: **02.02.2011**
(21) Numéro de dépôt: 08305393.4
(22) Date de dépôt: 10.07.2008
(51) Int. Cl.: A61B 3/09

(54) **Procédé et dispositif de détection d'une imprécision d'accommodation**
Verfahren und Vorrichtung zum Erkennen einer Ungenauigkeit bei der Akkommodation
Method and device for detecting an accommodation inaccuracy

(43) Date de publication de la demande: 13.01.2010
(73) Titulaire: Essilor International (Compagnie Générale D'Optique), 94220 Charenton le Pont (FR)
(72) Inventeur: Drobe, Bjorn, 94220, CHARENTON-LE-PONT (FR); Giraudet, Guillaume, 94220, CHARENTON-LE-PONT (FR)
(74) Mandataire: Lenne, Laurence

(56) Documents cités:
- DE-A1- 1 945 814
- US-A- 1 526 781
- US-A- 4 618 231
- US-A- 4 698 564
- US-A- 4 778 268

## Description

L'invention concerne un procédé et un dispositif de détection d'une imprécision d'accommodation.

Le mécanisme d'accommodation est le processus par lequel l'oeil réalise la mise au point sur un objet, par modification de la puissance du cristallin et/ou réduction du diamètre pupillaire. Une imprécision d'accommodation peut être de deux types.

Le retard accommodatif ou insuffisance d'accommodation (dit également « lag » accommodatif) est la différence négative entre la réponse accommodative de l'oeil et le stimulus dioptrique d'accommodation.

L'avance accommodative ou excès d'accommodation (dit également «lead» accommodatif) est la différence positive entre la réponse accommodative de l'oeil et le stimulus dioptrique d'accommodation.

Il existe des procédés et des dispositifs connus de détection de l'imprécision d'accommodation.

Cette détection peut être effectuée au moyen d'un autoréfracteur en vision de près à champ ouvert tel que le « Shin Nippon SRW 5000 » , le « Canon R-1 » ou « le Grand Seiko WAM 5500 ». Ces appareillages électroniques sont très complexes, encombrants et coûteux et nécessitent un utilisateur averti.

Il est également connu d'utiliser un skiascope en vision de près moins coûteux qu'un autoréfracteur, mais toujours relativement coûteux et nécessitant un utilisateur expert très entraîné et une manipulation relativement longue.

L'objet de l'invention est de proposer un procédé et un dispositif de détection de l'imprécision d'accommodation, peu coûteux, simples et pouvant être utilisés par une personne novice, non avertie.

Pour ce faire, l'invention propose un procédé de détection de l'imprécision d'accommodation d'un individu consistant
- à équiper au moins un de ses yeux, en plus de son éventuelle correction visuelle, d'un verre de lunette sphéro-cylindrique présentant un cylindre de prescription Cyl non nul dont la valeur absolue est comprise entre 0,50 et 2,00 dioptries,
- à faire fixer par cet oeil une première cible de fixation en vision de près,
- à lui présenter en vision de près durant un laps de temps compris entre 20 et 300 millisecondes un motif de détection comportant deux directions orthogonales privilégiées coïncidant avec l'axe et le contraxe du dit verre,
- à détecter une imprécision d'accommodation, si l'individu a perçu l'une des directions privilégiées avec une netteté différente.

L'accommodation étant une information centrale résultante de l'activité cérébrale, le procédé peut être appliqué de façon monoculaire ou binoculaire.

Selon un mode de réalisation préféré, ledit laps de temps est de l'ordre de 200 millisecondes.

De préférence, ledit verre présente un cylindre de prescription Cyl dont la valeur absolue est comprise entre 1,50 et 2,00 dioptries.

De préférence, ledit verre présente une valeur de sphère de prescription Sph telle que Sph+Cyl/2 soit égale à 0,00 dioptrie.

De préférence, ledit motif de détection est de taille angulaire supérieure à 0,5°.

De préférence, ledit motif de détection comprend un nombre de transitions abruptes de luminance dans chaque direction privilégiée inférieur ou égal à 60 transitions par degré d'angle visuel.

Avantageusement, ledit motif de détection est une croix de Jackson.

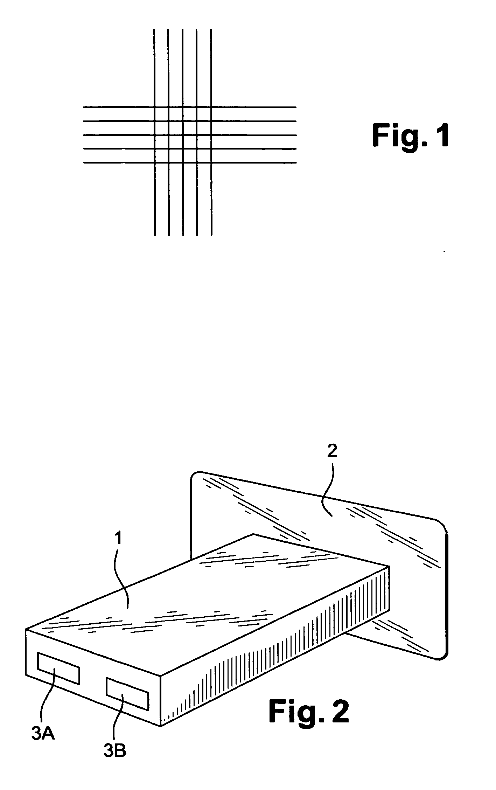

Il est connu d'utiliser le motif dit croix de Jackson en vision de loin, afin de vérifier une réfraction, selon la méthode subjective dite des cylindres croisés fixes. Cette méthode est décrite dans l'ouvrage « l'opticien Lunetier », Caroline Kovarski, 2005, P1039 à 1041. Cette méthode peut aussi être utilisée en vision de près afin de vérifier une addition prescrite. Toutefois, aucune contrainte de temps n'est imposée sur la présentation de la croix de Jackson, ce qui ne permet pas de contôler l'accommodation qui demeure fluctuante. Une croix dite de Jackson est ici représentée sur la figure 1. C'est un motif standardisé quant à l'épaisseur de ses lignes et à son contraste.

Ladite première cible de fixation peut représenter un point ou une croix de Malte.

Avantageusement la première cible de fixation est de taille angulaire inférieure à 0,1°, de préférence inférieure à 0,05°.

Avantageusement, la présentation du dit motif de détection est suivie de la présentation d'une seconde cible représentant un point ou une croix de Malte.

Ladite première cible de fixation peut être présentée durant au moins deux secondes.

De préférence, l'écart entre la distance de présentation de la première cible et celle du motif de détection est inférieur à 5 mm et, avantageusement, la distance de présentation de la première cible et celle du motif de détection sont identiques.

L'invention concerne également un dispositif de détection de l'imprécision d'accommodation d'un individu pour la mise en oeuvre de ce procédé, comportant un tachistoscope comportant un agencement de présentation d'une première cible de fixation en vision de près et d'un motif de détection comportant deux directions orthogonales privilégiées, également en vision de près, durant un laps de temps compris entre 20 et 300 milliseconde, selon la revendication 13.

De préférence, ledit tachistoscope comporte un moyen de présentation dudit motif dans un laps de temps de l'ordre de 200 millisecondes.

Ledit motif de détection est de taille angulaire supérieure à 0,5°

De préférence, ledit motif de détection comprend un nombre de transitions abruptes de luminance dans chaque direction privilégiée inférieur ou égal à 60 transitions par degré d'angle visuel.

Ledit motif de détection peut être une croix de Jackson.

Avantageusement, ladite première cible de fixation est de taille angulaire inférieure ou égale à 0,1°, de préférence inférieure ou égale à 0,05°.

Ladite première cible de fixation peut représenter un point ou une croix de Malte.

De préférence, ledit tachistoscope comporte un moyen de présentation finale d'une seconde cible représentant un point ou une croix de Malte.

Avantageusement, ledit tachistoscope comporte un moyen de présentation de ladite première cible de fixation dans un laps de temps d'au moins deux secondes.

De préférence, ledit tachistoscope comporte un boîtier pourvu, à l'une de ses extrémités, d'au moins une ouverture de regard et, à l'autre de ces extrémités, d'un agencement de présentation desdits cible(s) et motif(s).

Ladite ouverture de regard peut être pourvue d'un verre de lunette sphéro-cylindrique présentant un cylindre de prescription Cyl non nul dont la valeur absolue est comprise entre 0,50 et 2,00 dioptries et dont l'axe coïncide avec l'une des directions privilégiées du motif de détection.

Avantageusement, ledit verre présente un cylindre de prescription Cyl dont la valeur absolue est comprise entre 1,50 et 2,00 dioptries.

Avantageusement, ledit verre présente une sphère de prescription Sph telle que Sph+Cyl/2 soit égale à 0 D.

De préférence, l'écart entre la distance de présentation de la première cible et celle du motif de détection est inférieur à 5 mm.

Selon une première variante, ledit agencement de présentation est un écran d'ordinateur.

Selon une seconde variante, ledit agencement de présentation est constitué d'un fond dudit boîtier pourvu dudit motif de détection et d'un volet mobile permettant l'occultation ou la vision du dit motif de détection.

Avantageusement, ledit volet comporte une ouverture de visualisation du dit motif de détection et, de part et d'autre de cette ouverture, est constitué desdites première et seconde cibles.

Ledit volet peut être translatable sensiblement parallèlement au et à proximité dudit fond du boîtier.

Ledit volet peut être rotatif autour d'un axe sensiblement perpendiculaire dudit fond du boîtier et à proximité de ce dernier.

L'invention est décrite ci-après plus en détail à l'aide de figures ne représentant que des modes de réalisation préférés de l'invention.
La figure 1 déjà évoquée représente une croix de Jackson.
La figure 2 est une vue en perspective d'un dispositif conforme à l'invention selon une première variante.
Les figures 3 sont des vues en perspective ou en plan d'un dispositif conforme à l'invention selon une deuxième variante.
Les figures 4 sont des vues en perspective ou en plan d'un dispositif conforme à l'invention selon une troisième variante.
Les figures 5 sont des vues en perspective ou en plan d'un dispositif conforme à l'invention selon une troisième variante.

Conformément à l'invention, le procédé de détection d'une imprécision d'accommodation d'un individu consiste
- à équiper au moins un de ses yeux, en plus de son éventuelle correction visuelle, d'un verre de lunette sphéro-cylindrique présentant un cylindre de prescription Cyl non nul dont la valeur absolue est comprise entre 0,50 et 2,00 dioptries,
- à faire fixer par cet oeil une première cible de fixation en vision de près,
- à lui présenter en vision de près durant un laps de temps compris entre 20 et 300 millisecondes un motif de détection comportant deux directions orthogonales privilégiées coïncidant avec l'axe et le contraxe dudit verre,
- à détecter une imprécision d'accommodation, si l'individu a perçu l'une des directions privilégiées avec une netteté différente.

Ledit laps de temps est inférieur à 300 millisecondes, afin d'être inférieur au déclenchement du phénomène d'accommodation. L'accommodation n'a donc pas le temps de se modifier durant ce laps de temps.

Selon un mode de réalisation préféré, le verre présente un cylindre de prescription Cyl de valeur absolue comprise entre 1,50 et 2,00 dioptries et une valeur de sphère de prescription Sph telle que la valeur de Sph+Cyl/2 soit nulle, soit par exemple une sphère de +0,75 dioptrie et un cylindre de -1,50 dioptrie orienté à 0°, ledit laps de temps est de l'ordre de 200 millisecondes, et le motif de détection est une croix de Jackson présentée à une distance en vision de près correspondant environ à la longueur d'avant bras de la personne, et de taille angulaire d'environ 4,5 degrés, soit donc une hauteur de l'ordre de 2 à 3 cm.

Ce procédé suppose que le porteur ait une vue parfaitement corrigée avant son application afin que son amétropie sphérique ou cylindrique ne perturbe pas le résultat. Une mesure d'acuité visuelle peut être nécessaire avant de mettre en oeuvre le procédé.

Après la présentation de la croix de Jackson pendant 200 millisecondes, il est demandé au porteur s'il a perçu plus nettement les lignes verticales ou les lignes horizontales de la croix. Compte tenu du choix précédent d'orientation du cylindre, s'il a perçu les lignes horizontales de façon plus nette, un retard d'accommodation peut être détecté. S'il a perçu les lignes verticales de façon plus nette, une avance d'accommodation peut être détectée. S'il n'a pas perçu de différence de netteté, l'imprécision d'accommodation est trop faible pour être détectée.

En choisissant un cylindre différent, pivoté de 90°, le résultat de la détection est inversé.

La première cible de fixation présente un motif de fixation qui peut être un point ou une croix de Malte et elle est présentée durant au moins deux secondes, afin d'éviter des fluctuations de l'accommodation en vision de près. De préférence, la première cible de fixation est de taille angulaire inférieure à 0,1°, de préférence inférieure à 0,05°.

La présentation du motif de détection constitué de la croix de Jackson est suivie de la présentation d'une seconde cible qui peut être uniforme et vierge de motif ou représenter également un point ou une croix de Malte. Son but est de ne pas distraire le porteur, lors de sa réponse à la question « quelles sont les lignes les plus nettes ? ».

L'écart entre la distance de présentation de la première cible et celle du motif de détection est inférieur à 5 mm et, avantageusement, la distance de présentation de la première cible et celle du motif de détection est identique.

Il peut être envisagé de répéter le procédé pour assurer le résultat de la détection, tout en tenant compte cependant que le porteur ne doit pas être entraîné au procédé, ce qui influencerait sa réponse et pourrait fausser le résultat.

Il peut être aussi envisagé de répéter le procédé en modifiant la valeur de sphère Sph dudit verre, afin de décaler artificiellement la valeur du seuil de l'imprécision d'accommodation détectable. La répétition du procédé en modifiant ladite valeur de sphère Sph permet alors d'évaluer l'imprécision d'accommodation, lorsque ce dernier est compris entre deux valeurs de seuil différentes. Dans cette configuration, le porteur ne doit pas être entraîné au procédé, ce qui influencerait sa réponse et pourrait fausser le résultat.

Des mesures comparatives, réalisées sur quatorze sujets à l'aide d'un « Autoréfractor » de type « Grand Seiko WAM 5500 », ont montré que le dispositif suivant conforme à l'invention permettait de détecter une imprécision d'accommodation de valeur absolue supérieure à 0,86 dioptries. Ce dispositif était pourvu d'un verre sphéro-cylindrique présentant une sphère de +0,75 dioptrie et un cylindre de -1,50 dioptries orienté à 0°, un laps de temps de l'ordre de 200 millisecondes, et une croix de Jackson présentée à une distance en vision de près de 33 cm, et de taille angulaire d'environ 4,5 degrés.

Des dispositifs de mise en oeuvre de ce procédé vont maintenant être décrits.

De façon générale, un dispositif de détection d'une imprécision d'accommodation d'un individu, conforme à l'invention, comporte un tachistoscope comportant un agencement de présentation d'une première cible de fixation en vision de près et d'un motif de détection comportant au moins deux directions orthogonales privilégiées, également en vision de près durant un laps de temps compris entre 20 et 300 millisecondes, de préférence de 200 millisecondes.

La figure 2 représente un premier mode de réalisation conforme à l'invention.

Le tachistoscope comporte un boîtier 1 de type pupillomètre pourvu, à l'une de ses extrémités, d'au moins une ouverture de regard et, à l'autre de ces extrémités, d'un agencement de présentation desdits cible(s) et motif(s) 2. Le dispositif représenté est binoculaire et comporte donc deux ouvertures de regard 3A et 3B. L'agencement de présentation est ici un écran d'ordinateur disposé à une extrémité ouverte du boîtier 1 opposée à l'extrémité portant les ouvertures de regard. Un programme spécifique assure alors la présentation des cible(s) et motif(s) durant les laps de temps souhaités.

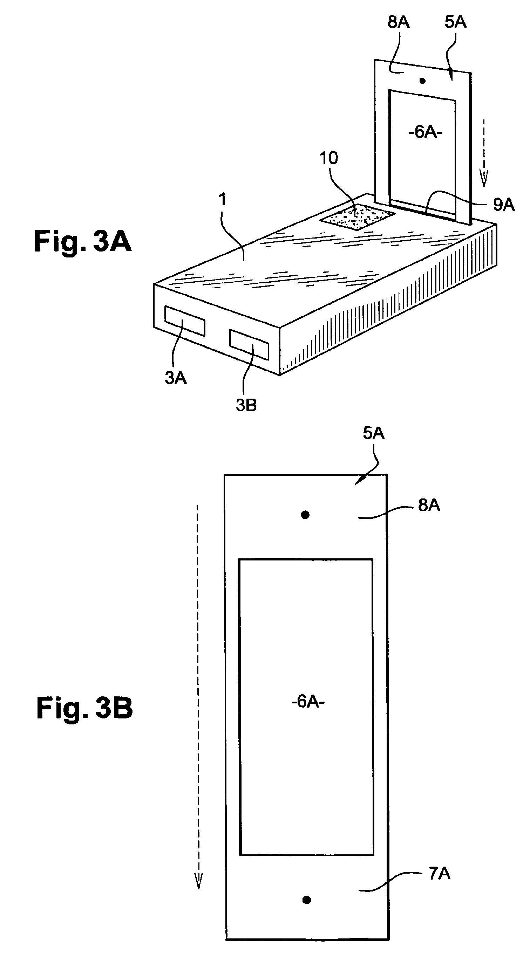

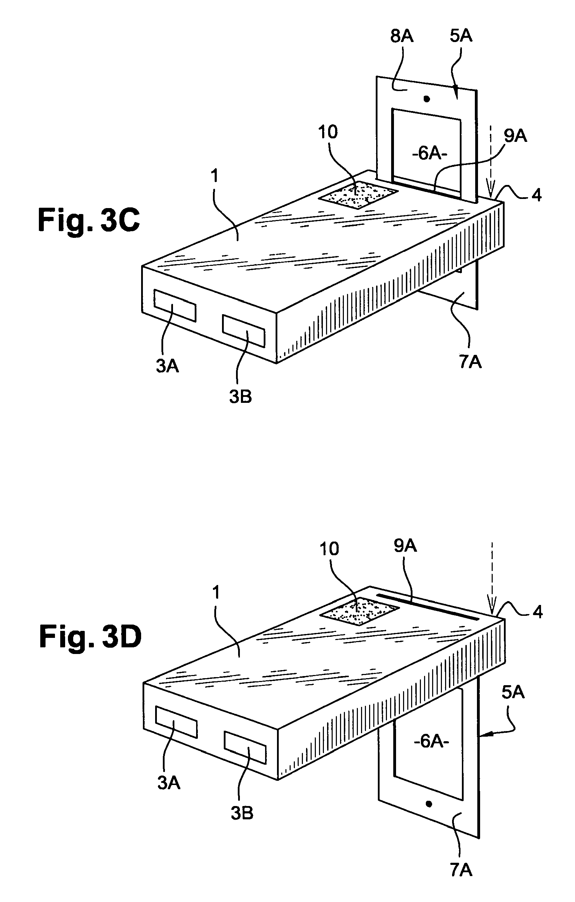

Les figures 3 représentent un deuxième mode de réalisation conforme à l'invention.

Le tachistoscope comporte un boîtier 1 pourvu, à l'une de ses extrémités, d'au moins une ouverture de regard et, à l'autre de ces extrémités, d'un agencement de présentation desdits cible(s) et motif(s). Le dispositif représenté est binoculaire et comporte donc deux ouvertures de regard 3A et 3B.

L'agencement de présentation est constitué d'un fond 4 du boîtier 1 opposé à l'extrémité portant les ouvertures de regard et ce fond 4 pourvu du motif de détection, de préférence une croix de Jackson, et d'un volet mobile 5A permettant l'occultation ou la vision de ce motif de détection.

Ce volet 5A comporte une ouverture de visualisation 6A du motif de détection et, de part et d'autre de cette ouverture, est constitué desdites première 7A et seconde 8A cibles. Le volet est translatable verticalement, sensiblement parallèlement au et à proximité du fond 4 du boîtier.

Plus précisément, le volet 5A est translaté dans les fentes 9A agencées sensiblement parallèlement au et à proximité du fond 4 du boîtier, dans la paroi supérieure et dans la paroi inférieure du boîtier.

La translation du volet 5A peut être réalisée par simple gravité, en maintenant le volet en position haute, c'est-à-dire avec la première cible 7A pourvue ici d'un point, visible par la personne, au moins deux secondes, ou être commandée par des moyens d'entraînement en soi connus, par exemple de type ressort, et actionnée par un bouton de commande 10. La taille de l'ouverture de visualisation est calculée en fonction des moyens d'entraînement pour obtenir le laps de temps souhaité de présentation du motif de détection, compris entre 20 et 300 millisecondes.

La seconde cible 8A représente ici également un point.

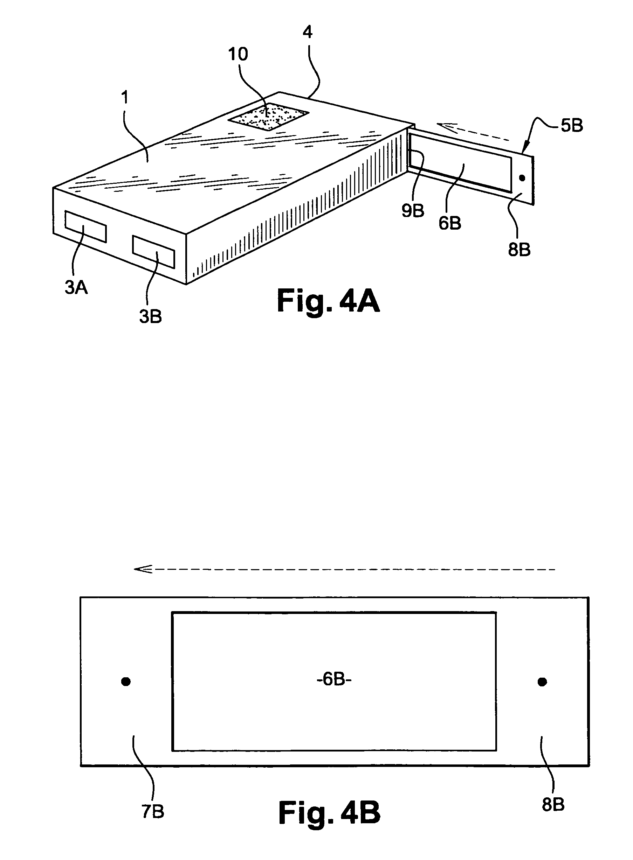

Les figures 4 représentent un troisième mode de réalisation conforme à l'invention.

Le tachistoscope comporte un boîtier 1 pourvu, à l'une de ses extrémités, d'au moins une ouverture de regard et, à l'autre de ces extrémités, d'un agencement de présentation desdits cible(s) et motif(s). Le dispositif représenté est binoculaire et comporte donc deux ouvertures de regard 3A et 3B.

L'agencement de présentation est constitué d'un fond 4 du boîtier 1 opposé à l'extrémité portant les ouvertures de regard et ce fond 4 pourvu du motif de détection, de préférence une croix de Jackson, et d'un volet mobile 5B permettant l'occultation ou la vision de ce motif de détection.

Ce volet 5B comporte une ouverture de visualisation 6B du motif de détection et, de part et d'autre de cette ouverture, est constitué desdites première 7B et seconde 8B cibles. Le volet est translatable horizontalement, sensiblement parallèlement au et à proximité du fond 4 du boîtier.

Plus précisément, le volet 5B est translaté dans les fentes 9B agencées sensiblement parallèlement au et à proximité du fond 4 du boîtier, dans les parois latérale du boîtier.

La translation du volet 5B peut être commandée par des moyens d'entraînement en soi connus, par exemple de type ressort, et actionnée par un bouton de commande 10. La taille de l'ouverture de visualisation est calculée en fonction des moyens d'entraînement pour obtenir le laps de temps souhaité de présentation du motif de détection, compris entre 20 et 300 millisecondes.

La seconde cible 8B représente ici également un point.

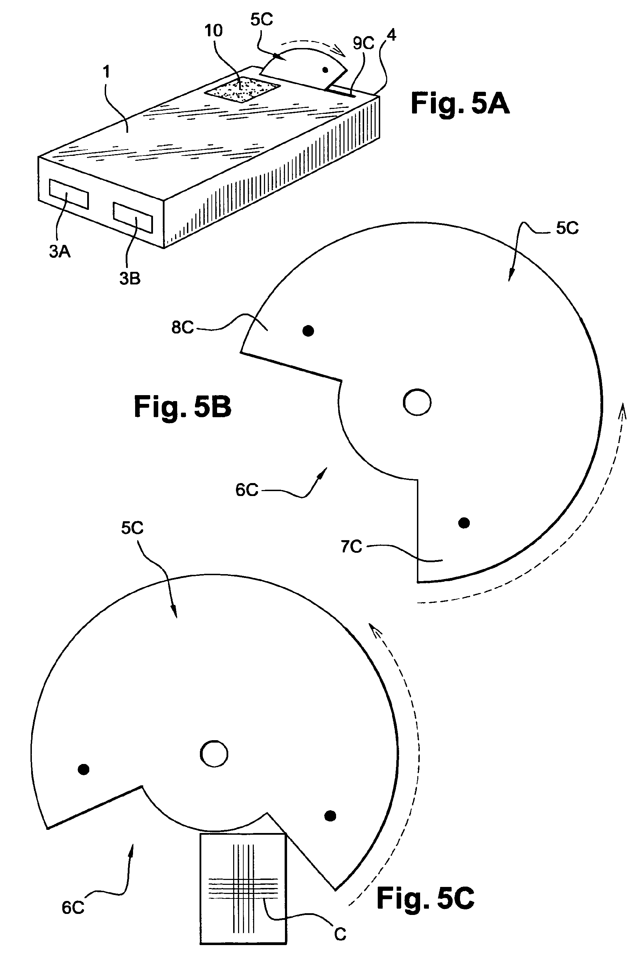

Les figures 5 représentent un quatrième mode de réalisation conforme à l'invention.

Le tachistoscope comporte un boîtier 1 pourvu, à l'une de ses extrémités, d'au moins une ouverture de regard et, à l'autre de ces extrémités, d'un agencement de présentation desdits cible(s) et motif(s). Le dispositif représenté est binoculaire et comporte donc deux ouvertures de regard 3A et 3B.

L'agencement de présentation est constitué d'un fond 4 du boîtier 1 opposé à l'extrémité portant les ouvertures de regard et ce fond 4 pourvu du motif de détection, de préférence une croix de Jackson, et d'un volet mobile 5C permettant l'occultation ou la vision de motif de détection.

Ce volet 5C particulièrement visible sur les figures 5B et 5C est un disque comportant une ouverture de visualisation 6C du motif de détection C et, de part et d'autre de cette ouverture, est constitué desdites première 7C et seconde 8C cibles. Cette ouverture de visualisation 6C correspond à un secteur périphérique du disque.

Ce volet 5C est monté rotatif autour d'un axe sensiblement perpendiculaire au fond 4 du boîtier et à proximité de ce dernier.

Plus précisément, le volet 5C effectue une rotation dans des fentes 9C agencées sensiblement parallèlement au et à proximité du fond 4 du boîtier, dans la paroi supérieure et la paroi inférieure du boîtier.

La rotation du volet 5C peut être commandée par des moyens d'entraînement en soi connus, par exemple de type ressort, et actionnée par un bouton de commande 10. La taille de l'ouverture de visualisation est calculée en fonction des moyens d'entraînement pour obtenir le laps de temps souhaité de présentation du motif de détection, compris entre 20 et 300 millisecondes.

La seconde cible 8C représente ici également un point.

L'invention ne se limite pas aux modes de réalisation illustrés et décrits et des variantes entrent dans le cadre de l'invention.

La première cible est importante, afin de diriger l'oeil de la personne dans une bonne direction où elle verra apparaître le motif de détection. Cette première cible permet aussi de donner une référence de proximité. Elle peut également représenter une croix de Malte de dimension équivalente au point illustré.

La seconde cible, quant à elle, a pour fonction de ne pas distraire la personne et peut être uniforme, vierge de motif, ou représenter une croix de Malte de dimension équivalente au point illustré.

Comme il a été déjà vu, le tachistoscope peut être binoculaire comme ici illustré ou être monoculaire.

La personne doit être équipée d'un verre de lunette sphéro-cylindrique présentant un cylindre de prescription Cyl non nul dont la valeur absolue est comprise entre 0,50 et 2,00 dioptries, de préférence égale à 1,50 dioptries.

Ce verre peut être disposé sur la personne au moyen un support indépendant comme une paire de lunettes par exemple.

De préférence, les ouvertures de regard 3A, 3B, sont pourvues de ce verre.

L'invention n'est pas limitée aux modes de réalisation préférés décrits.

Au lieu d'une croix de Jackson, il est possible d'utiliser un autre motif présentant deux directions orthogonales privilégiées comportant chacune au moins une transition abrupte entre deux niveaux de luminance. Le profil de luminance dans deux directions privilégiées est dit carré, ceci afin de pouvoir détecter plus facilement un léger flou lié à l'imprécision d'accommodation. Le profil de transition de luminance est alors perçu comme étant moins abrupte en présence d'un léger flou. Le nombre maximum de transitions abruptes de luminance présentées dans chaque direction privilégiée est inférieur ou égal à 60 transitions par degré d'angle visuel. Le contraste du motif est de préférence supérieur à 10%, préférentiellement supérieur à 50% et encore plus préférentiellement supérieur à 80%.

## Revendications

1. Procédé de détection de l'imprécision d'accommodation d'un individu consistant
- à équiper au moins un de ses yeux, en plus de son éventuelle correction visuelle, d'un verre de lunette sphéro-cylindrique présentant un cylindre de prescription Cyl non nul dont la valeur absolue est comprise entre 0,50 et 2,00 dioptries,
- à faire fixer par cet oeil une première cible de fixation (7A, 7B, 7C) en vision de près,
- à lui présenter en vision de près durant un laps de temps compris entre 20 et 300 millisecondes un motif de détection comportant deux directions orthogonales privilégiées coïncidant avec l'axe et le contraxe du dit verre,
- à détecter une imprécision d'accommodation, si l'individu a perçu l'une des directions privilégiées avec une netteté différente.

2. Procédé selon la revendication 1, **caractérisé en ce que** ledit laps de temps est de l'ordre de 200 millisecondes.

3. Procédé selon l'une des revendications précédentes, **caractérisé en ce que** ledit verre présente un cylindre de prescription Cyl dont la valeur absolue est comprise entre 1,50 et 2,00 dioptries.

4. Procédé selon l'une des revendications précédentes, **caractérisé en ce que** ledit verre présente une sphère de prescription Sph telle que Sph+Cyl/2 soit égale à 0 dioptrie.

5. Procédé selon l'une des revendications précédentes, **caractérisé en ce que** ledit motif de détection est de taille angulaire supérieure à 0,5°.

6. Procédé selon l'une des revendications précédentes, **caractérisé en ce que** ledit motif de détection comprend un nombre de transitions abruptes de luminance dans chaque direction privilégiée inférieur ou égal à 60 transitions par degré d'angle visuel.

7. Procédé selon l'une des revendications précédentes, **caractérisé en ce que** ledit motif de détection est une croix de Jackson (figure 1).

8. Procédé selon l'une des revendications précédentes, **caractérisé en ce que** ladite première cible de fixation (7A, 7B, 7C) représente un point ou une croix de Malte.

9. Procédé selon l'une des revendications précédentes, **caractérisé en ce que** ladite première cible de fixation (7A, 7B, 7C) est de taille angulaire inférieure ou égale à 0,1°.

10. Procédé selon l'une des revendications précédentes, **caractérisé en ce que** la présentation du dit motif de détection est suivie de la présentation d'une seconde cible (8A, 8B, 8C) représentant un point ou une croix de Malte.

11. Procédé selon l'une des revendications précédentes, **caractérisé en ce que** ladite première cible de fixation (7A, 7B, 7C) est présentée durant au moins deux secondes.

12. Procédé selon l'une des revendications précédentes, **caractérisé en ce que** l'écart entre la distance de présentation de la première cible et celle du motif d'évaluation est inférieure à 5 mm.

13. Dispositif de détection de l'imprécision d'accommodation d'un individu pour la mise en oeuvre du procédé selon l'une des revendications précédentes, **caractérisé en ce qu'**il comporte un tachistoscope comportant un boîtier (1) de longueur constante pourvu, à l'une de ses extrémités, d'au moins une ouverture de regard (3A, 3B) et, à l'autre de ces extrémités, d'un agencement de présentation d'une première cible de fixation (7A, 7B, 7C) en vision de près et d'un motif de détection comportant deux directions orthogonales privilégiées, également en vision de près, durant un laps de temps compris entre 20 et 300 millisecondes, et au moins un verre de lunette sphéro-cylindrique associé présentant un cylindre de prescription Cyl non nul dont la valeur absolue est comprise entre 0,50 et 2,00 dioptries et dont l'axe coïncide avec l'une des directions privilégiées du motif de détection.

14. Dispositif selon la revendication précédente, **caractérisé en ce que** ledit tachistoscope comporte un moyen de présentation dudit motif de détection dans un laps de temps de l'ordre de 200 millisecondes.

15. Dispositif selon la revendication 13 ou 14, **caractérisé en ce que** ledit tachistoscope comporte un moyen de présentation finale d'une seconde cible (8A, 8B, 8C) représentant un point ou une croix de Malte.

16. Dispositif selon l'une des revendications 13 à 15, **caractérisé en ce que** ledit tachistoscope comporte un moyen de présentation de ladite première cible de fixation (7A, 7B, 7C) dans un laps de temps d'au moins deux secondes.

17. Dispositif selon l'une des revendications précédente 13 à 16, **caractérisé en ce que** ladite ouverture de regard (3A, 3B) est pourvue dudit verre de lunette sphéro-cylindrique.

18. Dispositif selon l'une des revendications 13 à 17, **caractérisé en ce que** ledit agencement de présentation est un écran d'ordinateur (2).

19. Dispositif selon l'une des revendications 13 à 17, **caractérisé en ce que** ledit agencement de présentation est constitué d'un fond dudit boîtier (1) pourvu dudit motif de détection et d'un volet mobile (5A, 5B, 5C) permettant l'occultation ou la vision du dit motif de détection.

20. Dispositif selon la revendication précédente, **caractérisé en ce que** ledit volet (5A, 5B, 5C) comporte une ouverture de visualisation (6A, 6B, 6C) du dit motif de détection et, de part et d'autre de cette ouverture, est constitué desdites première et seconde cibles.

## Claims

1. A method for detecting the inaccuracy of accommodation of an individual, comprising:
- fitting at least one of the individual's eyes, in addition to his or her possible visual correction, with a spherical cylindrical lens having a non-zero prescription cylinder Cyl, the absolute value of which is comprised between 0.50 and 2.00 diopters,
- have this eye look fixedly at a first near vision fixation target (7A, 7B, 7C),
- showing it in near vision for a time interval comprised between 20 and 300 milliseconds a detection pattern having two privileged orthogonal directions coinciding with the axis and the counter-axis of said lens,
- detecting inaccuracy of accommodation, if the individual has perceived one of the privileged directions with different sharpness.

2. The method according to claim 1, **characterized in that** said time interval is on the order of 200 milliseconds.

3. The method according to any of the preceding claims, **characterized in that** said lens has a prescription cylinder Cyl, the absolute value of which is comprised between 1.50 and 2.00 diopters.

4. The method according to any of the preceding claims, **characterized in that** said lens has a prescription sphere Sph such that Sph+Cyl/2 is 0 diopters.

5. The method according to any of the preceding claims, **characterized in that** said detection pattern has an angular size greater than 0.5°.

6. The method according to any of the preceding claims, **characterized in that** said detection pattern comprises a number of sharp luminance transitions in each privileged direction less or equal to 60 transitions per visual angle degree.

7. The method according to any of the preceding claims, **characterized in that** said detection pattern is a Jackson cross (Fig. 1).

8. The method according to any of the preceding claims, **characterized in that** said first fixation target (7A, 7B, 7C) represents a dot or a Maltese cross.

9. The method according to any of the preceding claims, **characterized in that** said first fixation target (7A, 7B, 7C) has an angular size smaller or equal to 0.1°.

10. The method according to any of the preceding claims, **characterized in that** presenting said detection pattern is followed by presenting a second target (8A, 8B, 8C) representing a dot or a Maltese cross.

11. The method according to any of the preceding claims, **characterized in that** said first fixation target (7A, 7B, 7C) is presented for at least two seconds.

12. The method according to any of the preceding claims, **characterized in that** the deviation between the distance for presenting the first target and that of the evaluating pattern is smaller than 5 mm.

13. A device for detecting the inaccuracy of accommodation of an individual for implementing the method according to any of the preceding claims, **characterized in that** it comprises a tachistoscope having a constant length housing (1), provided at one of the ends thereof with at least one sight hole (3A, 3B) and, at the other one of the ends, with an arrangement for presenting a first near vision fixation target (7A, 7B, 7C) and a detection pattern having two privileged orthogonal directions, also in near vision, for a time interval comprised between 20 and 300 milliseconds, and at least one associated spherical cylindrical lens having a non-zero prescription cylinder Cyl, the absolute value of which is comprised between 0.50 to 2.00 diopters, and the axis of which coincides with one of the privileged directions of the detection pattern.

14. The device according to the preceding claim, **characterized in that** said tachistoscope has a means for presenting said detection pattern for a time interval on the order of 200 milliseconds.

15. The device according to claim 13 or claim 14, **characterized in that** said tachistoscope has a means for finally presenting a second target (8A, 8B, 8C) representing a dot or a Maltese cross.

16. The device according to any of claims 13 to 15, **characterized in that** said tachistoscope has a means for presenting said first fixation target (7A, 7B, 7C) for a time interval of at least two seconds.

17. The device according to any of the preceding claims 13 to 16, **characterized in that** said sight hole (3A, 3B) is provided with said spherical cylindrical lens.

18. The device according to any of claims 13 to 17, **characterized in that** said presenting arrangement is a computer screen (2).

19. The device according to any of claims 13 to 17, **characterized in that** said presenting arrangement consists of a bottom of said housing (1) provided with said detection pattern and a moving shutter (5A, 5B, 5C) allowing for said detection pattern to be hidden or visible.

20. The device according to the preceding claim, **characterized in that** said shutter (5A, 5B, 5C) comprises an aperture (6A, 6B, 6C) for viewing said detection pattern and, on either side of this aperture, consists of said first and second targets.

## Patentansprüche

1. Verfahren zum Erfassen der Akkommodationsungenauigkeit einer Person, umfassend folgende Schritte:
- Ausstatten mindestens eines der Augen der Person, zusätzlich zu ihrer eventuellen Sehkorrektur, mit einem sphärisch-zylindrischen Brillenglas, das einen Verschreibungszylinder Cyl ungleich Null aufweist, dessen absoluter Wert zwischen 0,50 und 2,00 Dioptrien liegt,
- dieses Auge eine erste Fixiermarke (7A, 7B, 7C) bei Nahsicht fixieren Lassen,
- dem Auge bei Nahsicht während eines Zeitraums von 20 bis 300 Millisekunden ein Erfassungsmuster Vorlegen, das zwei bevorzugte orthogonale Richtungen aufweist, die mit der Achse und der Gegenachse des Brillenglases zusammenfallen,
- Erfassen einer Akkommodationsungenauigkeit, wenn die Person eine der bevorzugten Richtungen mit einer anderen Schärfe wahrgenommen hat.

2. Verfahren nach Anspruch 1, **dadurch gekennzeichnet, dass** der Zeitraum ungefähr 200 Millisekunden lang ist.

3. Verfahren nach einem der vorhergehenden Ansprüche, **dadurch gekennzeichnet, dass** das Brillenglas einen Verschreibungszylinder Cyl aufweist, dessen absoluter Wert zwischen 1,5 und 2,00 Dioptrien beträgt.

4. Verfahren nach einem der vorhergehenden Ansprüche, **dadurch gekennzeichnet, dass** das Brillenglas eine derartige Verschreibungssphäre Sph aufweist, dass Sph+Cyl/2 gleich 0 Dioptrien ist.

5. Verfahren nach einem der vorhergehenden Ansprüche, **dadurch gekennzeichnet, dass** das Erfassungsmuster eine Winkelgröße aufweist, die größer als 0,5° ist.

6. Verfahren nach einem der vorhergehenden Ansprüche, **dadurch gekennzeichnet, dass** das Erfassungsmuster eine Anzahl von sprunghaften Luminanzübergängen in jeder bevorzugten Richtung umfasst, die sich auf weniger oder gleich 60 Übergänge pro Sichtwinkelgrad belaufen.

7. Verfahren nach einem der vorhergehenden Ansprüche, **dadurch gekennzeichnet, dass** das Erfassungsmuster ein Jackson-Kreuz ist (Fig. 1).

8. Verfahren nach einem der vorhergehenden Ansprüche, **dadurch gekennzeichnet, dass** die erste Fixiermarke (7A, 7B, 7C) einen Punkt oder ein Malteserkreuz darstellt.

9. Verfahren nach einem der vorhergehenden Ansprüche, **dadurch gekennzeichnet, dass** die erste Fixiermarke (7A, 7B, 7C) eine Winkelgröße aufweist, die kleiner oder gleich 0,1° ist.

10. Verfahren nach einem der vorhergehenden Ansprüche, **dadurch gekennzeichnet, dass** auf das Vorlegen des Erfassungsmusters das Vorlegen einer zweiten Marke (8A, 8B, 8C) folgt, die einen Punkt oder ein Malteserkreuz darstellt.

11. Verfahren nach einem der vorhergehenden Ansprüche, **dadurch gekennzeichnet, dass** die erste Fixiermarke (7A, 7B, 7C) mindestens zwei Sekunden lang vorgelegt wird.

12. Verfahren nach einem der vorhergehenden Ansprüche, **dadurch gekennzeichnet, dass** die Abweichung zwischen dem Abstand zum Vorlegen der ersten Marke und dem des Bewertungsmusters kleiner als 5 mm ist.

13. Vorrichtung zum Erfassen der Akkommodationsungenauigkeit einer Person zum Umsetzen des Verfahrens nach einem der vorhergehenden Ansprüche, **dadurch gekennzeichnet, dass** sie ein Tachistoskop umfasst, das ein Gehäuse (1) konstanter Länge, das an einem seiner Enden mit mindestens einem Schauloch (3A, 3B) und an dem anderen dieser Enden mit einer Anordnung zum Vorlegen einer ersten Nahsichtfixiermarke (7A, 7B, 7C) und eines Erfassungsmusters, das zwei bevorzugte orthogonale Richtungen aufweist, ebenfalls bei Nahsicht, während eines Zeitraums zwischen 20 und 300 Millisekunden versehen ist, und mindestens ein dazugehöriges sphärischzylindrisches Brillenglas, das einen Verschreibungszylinder Cyl ungleich Null aufweist, dessen absoluter Wert zwischen 0,50 und 2,00 Dioptrien beträgt und dessen Achse mit einer der bevorzugten Richtungen des Erfassungsmusters zusammenfällt, umfasst.

14. Vorrichtung nach dem vorhergehenden Anspruch, **dadurch gekennzeichnet, dass** das Tachistoskop ein Mittel aufweist, um das Erfassungsmuster während eines Zeitraums von ungefähr 200 Millisekunden vorzulegen.

15. Vorrichtung nach Anspruch 13 oder 14, **dadurch gekennzeichnet, dass** das Tachistoskop ein Mittel aufweist zum endgültigen Vorlegen einer zweiten Marke (8A, 8B, 8C), die einen Punkt oder ein Malteserkreuz darstellt.

16. Vorrichtung nach einem der Ansprüche 13 bis 15, **dadurch gekennzeichnet, dass** das Tachistoskop ein Mittel aufweist, um die erste Fixiermarke (7A, 7B, 7C) während eines Zeitraums von mindestens zwei Sekunden vorzulegen.

17. Vorrichtung nach einem der vorhergehenden Ansprüche 13 bis 16, **dadurch gekennzeichnet, dass** das Schauloch (3A, 3B) mit dem sphärisch-zylindrischen Brillenglas versehen ist.

18. Vorrichtung nach einem der Ansprüche 13 bis 17, **dadurch gekennzeichnet, dass** die Vorlegungsanordnung ein Computerbildschirm (2)ist.

19. Vorrichtung nach einem der Ansprüche 13 bis 17, **dadurch gekennzeichnet, dass** die Vorlegungsanordnung aus einem Boden des Gehäuses (1) besteht, der mit dem Erfassungsmuster und einem beweglichen Blendenschieber (5A, 5B, 5C) versehen ist, der es ermöglicht, das Erfassungsmuster zu maskieren oder sichtbar zu machen.

20. Vorrichtung nach dem vorhergehenden Anspruch, **dadurch gekennzeichnet, dass** der Blendenschieber (5A, 5B, 5C) eine Öffnung (6A, 6B, 6C) umfasst zum Sehen des Erfassungsmusters und auf beiden Seiten dieser Öffnung aus der ersten und der zweiten Marke besteht.
